# EUROPEAN PATENT APPLICATION

(11) **EP 1 161 956 A1**
(43) Date of publication of application: **12.12.2001**
(21) Application number: 00909677.7
(22) Date of filing: 16.03.2000
(51) Int. Cl.: A61K 47/10, A61K 47/30, A61K 47/44, A61K 9/14, A61K 31/7048, A61K 31/554, A61K 31/5415, A61K 31/704, A61K 31/52, A61K 31/522, A61K 31/4402, A61K 31/426, A61K 31/5383, A61K 31/4365

(54) **MEDICINAL COMPOSITIONS**

(30) Priority: 17.03.1999 JP 7214599
(71) Applicant: DAIICHI PHARMACEUTICAL CO., LTD., Chuo-ku, Tokyo 103-8234 (JP)
(72) Inventor: NAKAGAMI, Hiroaki Daiichi Pharmaceutical Co., Ltd., Tokyo 134-8630 (JP); SUZUKI, Tatsuya Daiichi Pharmaceutical Co., Ltd., Tokyo 134-8630 (JP); KOBAYASHI, Hideo Daiichi Pharmaceutical Co., Ltd., Tokyo 134-8630 (JP); KUROSAWA, Akira Daiichi Pharmaceutical Co., Ltd., Tokyo 134-8630 (JP)
(74) Representative: Hartz, Nikolai F., Dr.
(86) International application number: JP0001606
(87) International publication number: WO0054811

(57) **Abstract**

The present invention relates to a granular pharmaceutical composition comprising a drug having a disagreeable taste, a wax and a sugar alcohol; a method for preparing the same; and a pharmaceutical product for oral administration, comprising the granular composition. The product excellently masks a disagreeable taste possessed by a drug and provides good sensation upon oral administration, and therefore is easily ingested by even the elderly, children, and patients suffering dysphagia. Moreover, the product is suitable for administration using tube.

## Description

### Field of the Invention

The present invention relates to a granular pharmaceutical composition which masks a disagreeable taste of a drug and which provides favorable sensation upon oral administration, and to a pharmaceutical product prepared therefrom.

### Background Art

Oral administration of a drug having a disagreeable taste tends to decrease patient compliance and, in many cases, results in poor attainment of expected therapeutic effect.

Known methods for masking a disagreeable taste of fine granules drugs include a spray-coating method making use of water-insoluble polymers and methods making use of microencapsulation or addition of sweetening agents. An example spray-coating method making use of water-insoluble polymers is used to produce a sustained-release drug disclosed in Japanese Patent Application Laid-Open (*kokai*) No. 30709/1987, in which drug-containing nuclei are coated with ethylcellulose, and the release rate of the drug can be controlled by varying the thickness of an ethylcellulose coating. However, the technique disclosed therein is directed to sustained-release drugs, and does not provides a technique used for rapid-release drugs which have ability to mask a disagreeable taste. Drugs coated with a water-insoluble polymer impart a gritty taste to the mouth of the patient upon oral administration, and cause pain when caught between the patient's dentures, thus posing problems related to ease of administration. The microencapsulating method has drawbacks in that it makes the production procedure complicated due to use of organic solvents, and involves low yield and high production costs. The method using addition of sweetening agents provides poor masking effect for drugs having strong disagreeable taste.

Japanese Patent Application Laid-Open (*kokai*) No. 242568/1995 discloses granules drugs obtained by fusing with heat a hydrophobic substance having a melting point of 45-90°C and a surfactant, dissolving or suspending a drug having a disagreeable taste and a channeling agent, and granulating the resultant mixture by spray-granulation. In this publication, the surfactant and the channeling agent are incorporated for the purpose of increasing the elution rate of the drug, and they are respectively contained in amounts of 5-35% in the composition. However, surfactants are preferably used in reduced amounts from the viewpoint of safety. Also, in consideration of processing for forming the pharmaceutical products, spray-granulated products desirably contain smaller amounts of additives so as to allow other additives to be incorporated in increased amounts. Therefore, the surfactant and channeling agent are advantageously employed in amounts as small as possible. Japanese Patent Application Laid-Open (*kokai*) No. 267850/1995 discloses a pharmaceutical composition obtained by mixing one or several species of a drug having a disagreeable taste, one or several species of a water-soluble polymer, and one or several species of a wax; heating; and granulating the fused wax together with the drug(s) and water-soluble polymer(s). In this publication, water-soluble polymers are added for the same purpose as above; i.e., for increasing the dissolution rate of the drugs. The water-soluble polymers are incorporated in the pharmaceutical composition in amounts of 5-60%. For the same reasons as mentioned above, water-soluble polymers are preferably not used at all, or used in amounts as small as possible.

Solid granules, *inter alia,* powder products, preferably have good administration using tube adaptability, in addition to the aforementioned ability of masking unpleasant tastes. "Administration using tube" refers to a manner of administration which is suitably applied to patients who have difficulty in swallowing pharmaceutical products. According to administration using tube, a powder product is dispersed in water, and then the dispersion is transferred to a syringe for administering the dispersion to a patient through a tube inserted through the patient's nose or abdomen to the digestive tract. In most cases, the dispersion is prepared immediately before use. Therefore, it is required that the powder product be dispersed uniformly in a short period of time, and should not plug in the syringe or tube. Powder products which are coated with a pH-dependent polymer such as methacrylic acid copolymer cohere in a non-electrolyte liquid such as purified water or glucose solution, resulting in clogging in the syringe or tube. Therefore; such powders are not suitable for administration using tube. Similarly, powder products which are formed by use of a sugar serving as an excipient, such as lactose, also cause clogging in the syringe or tube, and thus are not suitable for administration using tube.

In view of the foregoing, an object of the present invention is to provide a granular pharmaceutical composition having excellent ability to mask a disagreeable taste of a drug and providing favorable sensation upon oral administration. Another object of the present invention is to provide a pharmaceutical product containing the same.

### Disclosure of the Invention

The present inventors have produced a granular product containing a drug having a disagreeable taste and have conducted extensive studies on the properties of the product. Surprisingly, the inventors have found that incorporating a sugar alcohol into a combination of a drug having a disagreeable taste and wax substance can provide a granular pharmaceutical product having excellent ability to mask a disagreeable taste and providing favorable sensation upon oral administration, leading to completion of the invention. The inventors have also found that the pharmaceutical product is available for administration using tube.

Accordingly, in a first aspect of the present invention, there is provided a granular pharmaceutical composition containing a drug having a disagreeable taste, a wax substance, and a sugar alcohol. In a second aspect of the present invention, there is provided a method for producing the granular pharmaceutical composition. In a third aspect of the present invention, there is provided a pharmaceutical product for oral administration containing the granular pharmaceutical composition.

### Best Modes for Carrying Out the Invention

In the present invention, the term "disagreeable taste" refers to any of a bitter taste, an astringent effect, a pungent taste, a disagreeable stimulation, and a disagreeable odor.

No particular limitation is imposed on the drug having a disagreeable taste so long as the drug provides the above-described taste and is used as a pharmaceutical. Examples of the drug include cetraxate hydrochloride, ecapapide, nefiracetam, talampicillin hydrochloride, indenolol hydrochloride, hydralazine hydrochloride, chlorpromazine hydrochloride, tiaramide hydrochloride, berberine chloride, digitoxin, sulpyrine, azelastine hydrochloride, etilefrine hydrochloride, diltiazem hydrochloride. propranolol hydrochloride. chloramphenicol, aminophyllin, erythromycin, clarithromycin, phenobarbital, calcium pantothenate, indeloxazine hydrochloride, aminoguanidine hydrochloride, bifemelane hydrochloride, 7β-[2-(2-aminothiazol-4-yl)-2-(Z)-hydroxyiminoacetamido]-3-N,N-dimethylcarb amoyloxymethyl-3-cephem-carboxylic acid 1-(isopropoxycarbonyloxy)ethyl ester hydrochloride, (E)-3-(2-methoxy-3,6-dimethyl-1,4-benzoquinon-5-yl)-2-[5-(3-pyridyl)pentyl]-2-propenic acid, aminophylline, theophylline, diphenhydramine, metoclopramide, phenylbutazone, phenobarbital, ampicillin, cimetidine, famotidine, nizatidine, acetaminophen, epirizole, pyrazinamide, caffeine, ethionamide, carvedilol, ranitidine hydrochloride, roxatidine acetate hydrochloride, imipramine hydrochloride, ephedrine hydrochloride, diphenhydramine hydrochloride, tetracycline hydrochloride, doxycycline hydrochloride, naphazoline hydrochloride. noscapine hydrochloride. papaverine hydrochloride, dextromethorphan hydrobromide, timepidium bromide, chlorphenilammonium maleate, alimemazine tartrate, pilsicainide hydrochloride, N-metylscopolamine methylsulfate, cinepazide maleate, arginine hydrochloride, histidine hydrochloride, lysine hydrochlroride, lysine acetate; crude drugs or extracts thereof such as Corydalis Tuber, Phellodendron Bark, Coptis Rhizome, Nux Vomica, Ephedra Herb, Ipecac, Scopolia Rhizome, Belladonna or Sophora Root; pyrridonecarboxylic acid compounds represented by formulas (1) through (4) and salts thereof: 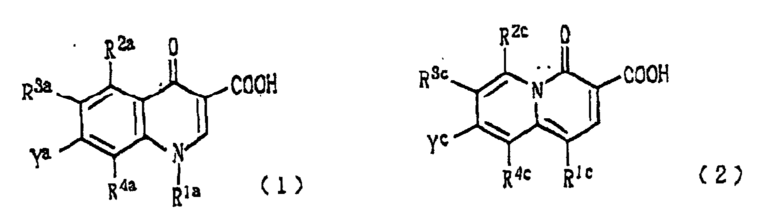 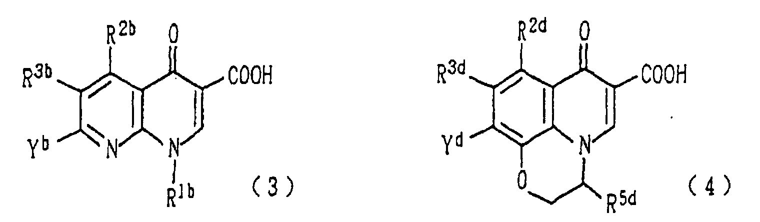
(wherein each of R^{1a}, R^{1b}, and R^{1c} represents a C1-C6 linear or branched alkyl group which may have a substituent, a C3-C6 cyclic alkyl group which may have a substituent, an aryl group which may have a substituent, or a heteroaryl group which may have a substituent;
each of R^{2a}, R^{2b}, R^{2c}, and R^{2d} represents a hydrogen atom or a C1-C6 linear or branched alkyl group which may have a substituent or an amino group;
each of R^{3a}, R^{3b}, R^{3c}, and R^{3d} represents a hydrogen atom or a halogen atom; R^{4a} or R^{4c} represents a hydrogen atom, a halogen atom, a C1-C6 linear or branched alkyl group which may have a substituent; or a C1-C6 linear or branched alkoxyl group which may have a substituent;
R^{5d} represents a hydrogen atom or a C1-C6 linear or branched alkyl group which may have a substituent; and
each of Y^{a}, Y^{b}, Y^{c}, and Y^{d} represents a nitrogen-containing group); and 4,5,6,7-tetrahydrothieno[3,2-c]pyridines or salts thereof represented by formula (5):

   R¹-CH(R²)-R³ (5)

[wherein R¹ represents a phenyl group which may have 1 to 3 substituents selected from among a C1-C4 alkyl group, a halogen atom, a fluorine-substituted C1-C4 alkyl group, C1-C4 alkoxyl group, a fluorine-substituted C1-C4 alkoxyl group, a cyano group, and a nitro group;
R² represents a hydrogen atom, a carboxyl group, a C1-C6 alkoxycarbonyl group, or a C1-C7 aliphatic acyl group which may have a substituent selected from among a halogen atom, a hydroxyl group, a C1-C4 alkoxyl group, and a cyano group; and
R³ represents a 4,5,6,7-tetrahydrothieno[3,2-c]pyridin-5-yl group which may have a substituent selected from among a hydroxyl group, a C1-C4 alkoxyl group, a C1-C4 alkoxyl group which may be substituted by C1-C4 alkoxyl or C1-C6 alkanoyloxy, a C7-C14 aralkyloxy group, a C1-C18 alkanoyloxy group, a C3-C7 cycloalkylcarbonyloxy group, a C6-C10 arylcarbonyloxy group, a C1-C4 alkoxycarbonyloxy group, and a C7-C14 aralkyloxycarbonyloxy group].

The above-described pyrridonecarboxylic acid compounds represented by formulas (1), (2), (3), or (4) and salts thereof and a method for producing the same are described in the following references: Japanese Patent Application Laid-Open (*kokai*) Nos. 53-141286, 55-31042, 57-46986, 57-77683, 60-36482, 60-64979, 60-228479, 62-252772, 62-252790, 62-277362, 1-230558, 1-258666, 1-294680, 2-28178, 2-124873, 2-231475, 5-271229, 7-309864, 8-41050 and WO 91/02526, WO 94/14794, WO 94/15933, WO 95/5373, WO 96/37475, WO 96/39407, WO 97/29102, WO 97/19072, WO 97/40037; WO 98/02431, WO 98/13370, WO 98/18783, WO 98/24781, WO 98/52939, WO 98/54169, or WO 98/58923. These publications also disclose production methods of the compounds and salts. The compounds represented by formula (5) and salts thereof may be produced by a method described in Japanese Patent Application Laid-Open (*kokai*) Nos. 50-46688, 58-10583, 59-27895, and 6-41139.

Any of the above-described compounds represented by formulas (1), (2), (3), (4), or (5) may have an asymmetric carbon atom and may exist as an optical isomer or a diastereomer. Such isomers *per se*, arbitrary mixtures thereof, racemic species, etc. are encompassed within the scope of the present invention. The above-described compounds represented by formulas (1) through (5) may exist as salts thereof, hydrates thereof, or solvates thereof, which are also included within the scope of the present invention.

In view of masking effect, the drug having a disagreeable taste is preferably slightly soluble in a wax; more preferably, soluble in water and slightly soluble in a wax.

Among the above-described compounds represented by formulas (1) through (4) and salts thereof, examples of preferred compounds include the following:

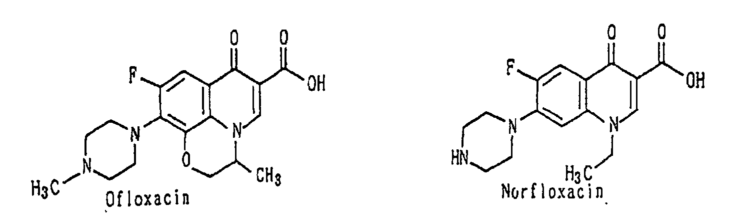

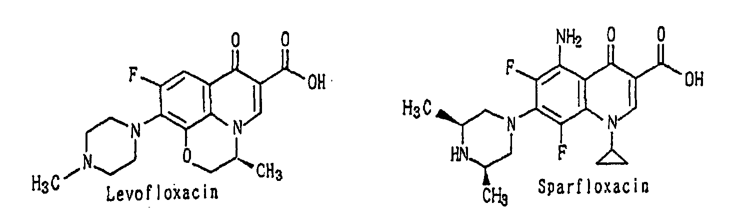

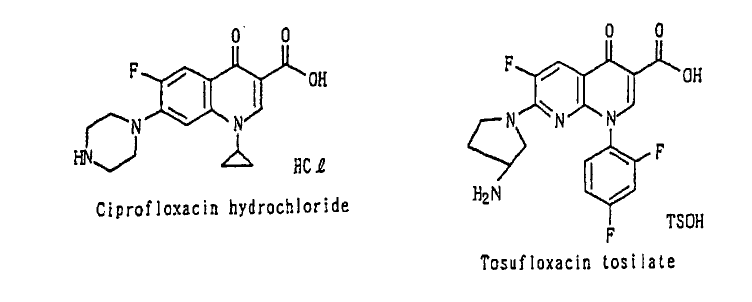

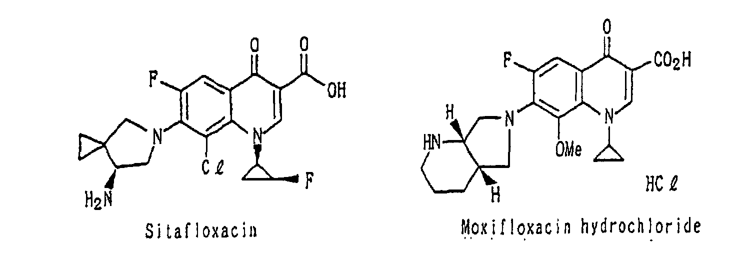

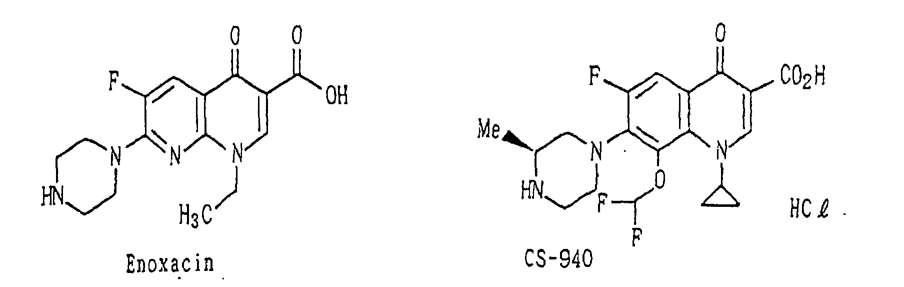

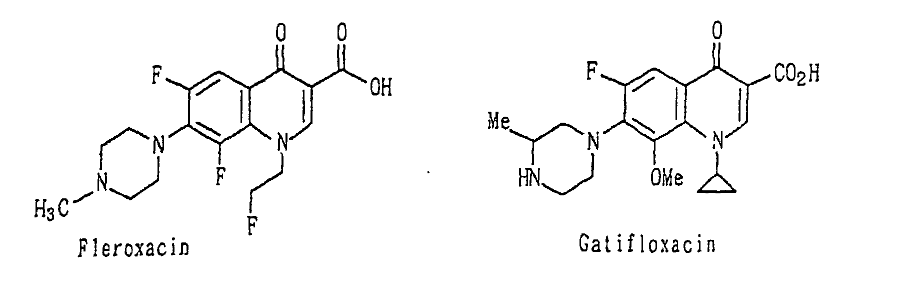

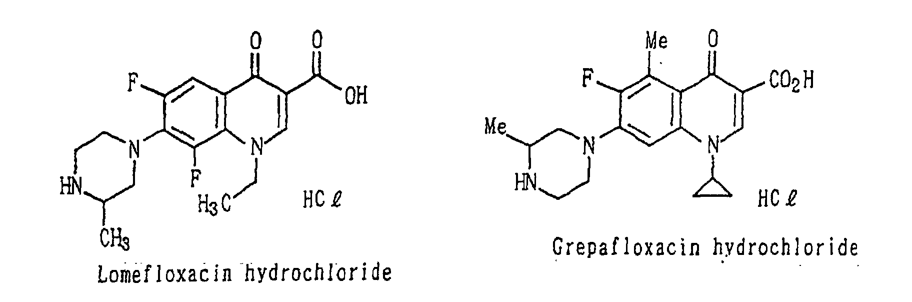

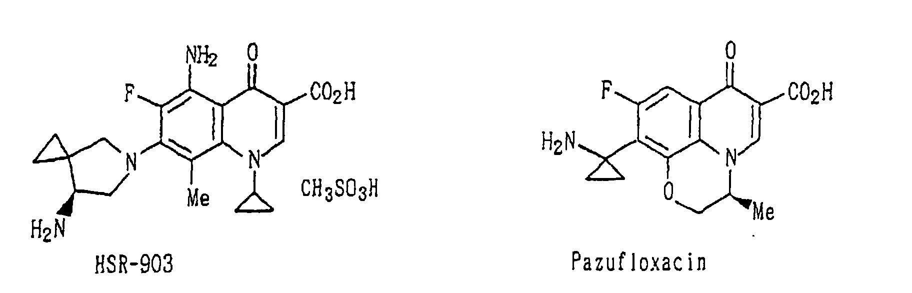

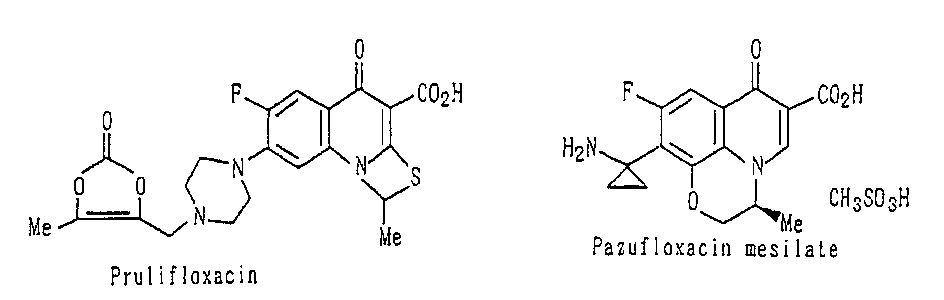

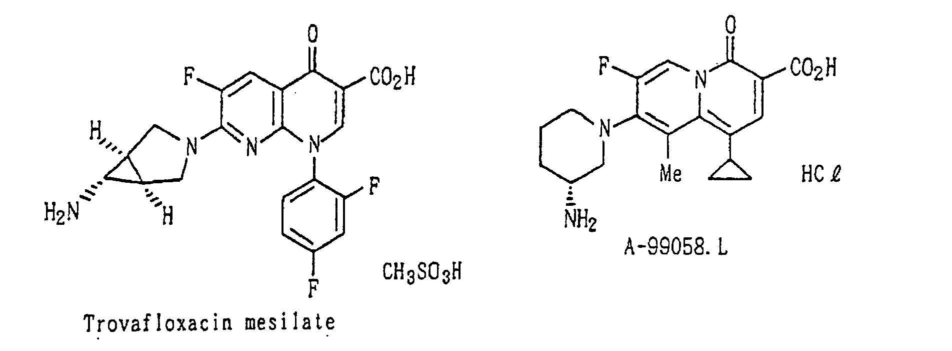

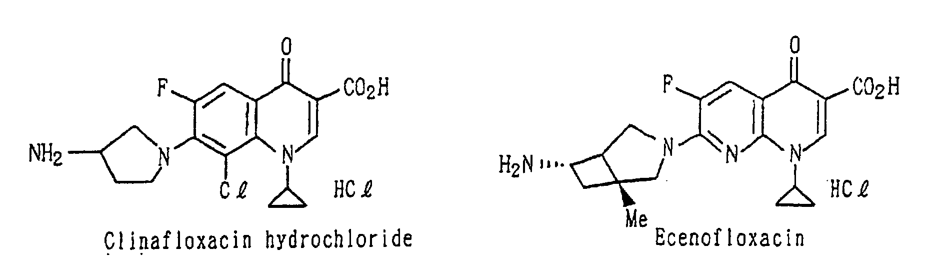

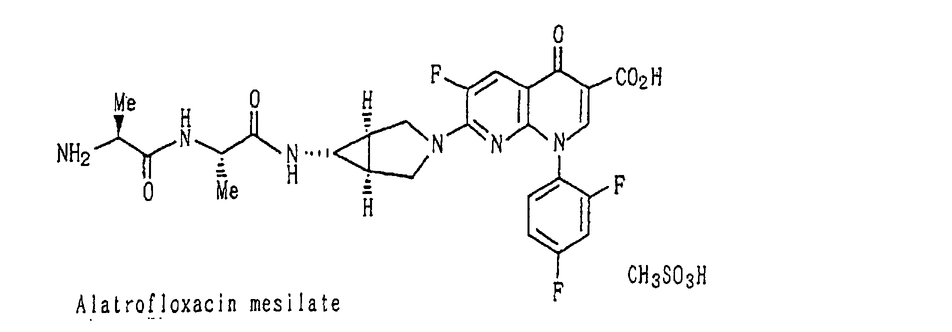

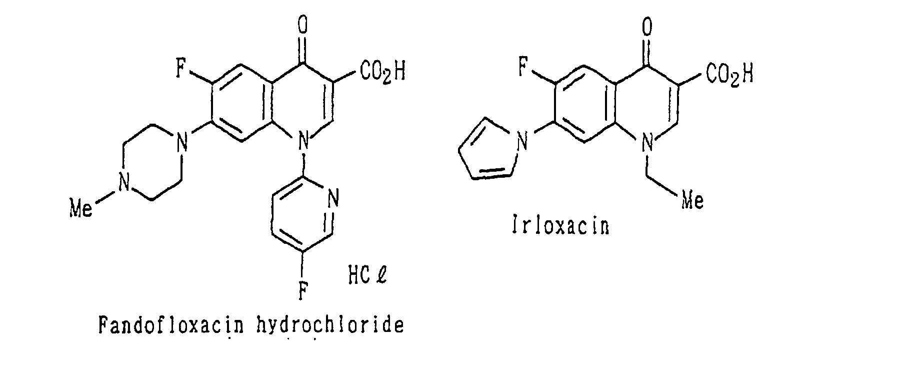

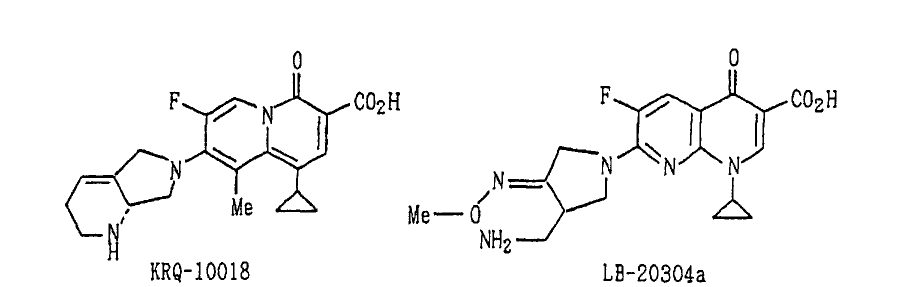

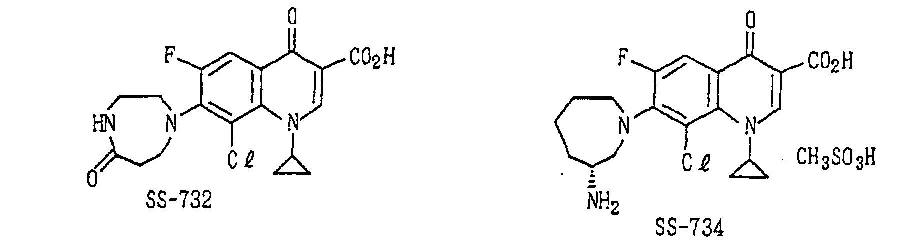

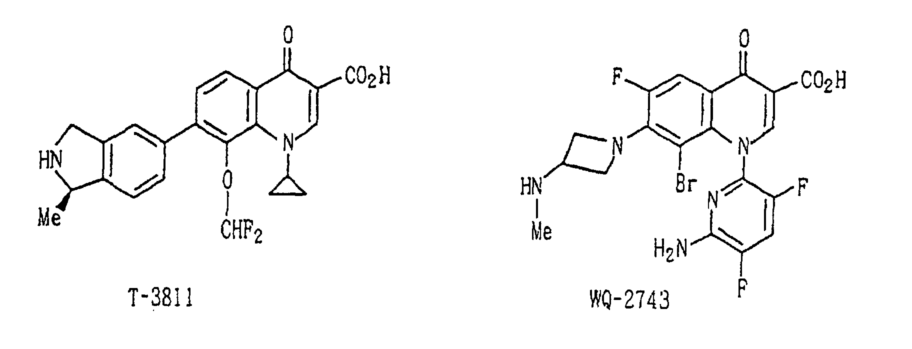

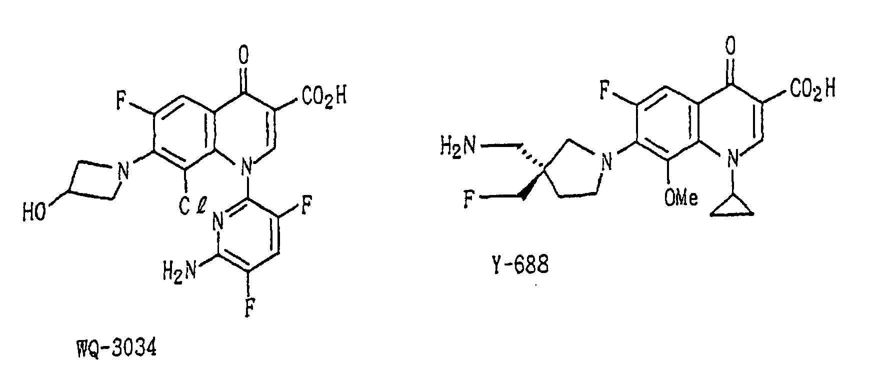

Also, among the compounds represented by formula (5) and salts thereof, examples of preferred compounds include the following:
2-hydroxy-5-(α-cyclopropylcarbonyl-2-chlorobenzyl)-4,5,6,7-tetrahydrothieno[3, 2-c]pyridine,
2-hydroxy-5-(α-propionyl-2-fluorobenzyl)-4,5,6,7-tetrahydrothieno [3,2-c]pyridine,
2-hydroxy-5-(α-cyclopropylcarbonyl-2-fluorobenzyl)-4,5,6,7-tetrahydrothieno[3,2-c]pyridine,
2-acetoxy-5-(α-cyclopropylcarbonyl-2-fluorobenzyl)-4,5,6,7-tetrahydrothieno[3,2-c]pyridine,
2-propionyloxy-5-(α-cyclopropylcarbonyl-2-fluorobenzyl)-4,5,6,7-tetrahydrothieno[3,2-c]pyridine,
2-butyryloxy-5-(α-cyclopropylcarbonyl-2-fluorobenzyl)-4,5,6,7-tetrahydrothieno [3,2-c]pyridine,
2-pivaloyloxy-5-(α-cyclopropylcarbonyl-2-fluorobenzyl)-4,5,6,7-tetrahydrothieno[3,2-c]pyridine,
2-valeryloxy-5-(α-cyclopropylcarbonyl-2-fluorobenzyl)-4,5,6,7-tetrahydrothieno[3,2-c]pyridine,
2-hexanoyloxy-5-(α-cyclopropylcarbonyl-2-fluorobenzyl)-4,5,6,7-tetrahydrothieno[3,2-c]pyridine,
2-t-butoxycarbonyloxy-5-(α-cyclopropylcarbonyl-2-fluorobenzyl)-4,5,6,7-tetrahydrothieno[3,2-c]pyridine,
2-pivaloyloxymethoxy-5-(α-cyclopropylcarbonyl-2-fluorobenzyl)-4,5,6,7-tetrahydrothieno[3,2-c]pyridine,
5-(α-cyclopropylcarbonyl-2-chlorobenzyl)-2-oxo-2,4,5,6,7,7a-hexahydrothieno[3,2-c]pyridine,
5-(α-propionyl-2-fluorobenzyl)-2-oxo-2,4,5,6,7,7a-hexahydrothieno[3,2-c]pyridine,
5-(α-cyclopropylcarbonyl-2-fluorobenzyl)-2-oxo-2,4,5,6,7,7a-hexahydrothieno[3, 2-c]pyridine,
2-acetoxy-5-(α-cyclopropylcarbonyl-2-chlorobenzyl)-4,5,6,7-tetrahydrothieno[3, 2-c]pyridine,
2-hydroxy-5-(a-2-fluorocyclopropylcarbonyl-2-fluorobenzyl)-4,5,6,7-tetrahydrothieno[3,2-c]pyridine,
5-(α-2-fluorocyclopropylcarbonyl-2-fluorobenzyl)-2-oxo-2,4,5,6,7,7a-hexahydrothieno[3,2-c]pyridine,
2-acetoxy-5-(α-2-fluorocyclopropylcarbonyl-2-fluorobenzyl)-4,5,6,7-tetrahydrothieno[3,2-c]pyridine,
5-(α-methoxycarbonyl-2-chlorobenzyl)-2-oxo-2,4,5,6,7,7a-hexahydrothieno[3,2-c]pyridine,
2-acetoxy-5-(α-methoxycarbonyl-2-chlorobenzyl)-4,5,6,7-tetrahydrothieno[3,2-c] pyridine,
5-(α-methoxycarbonyl-2-fluorobenzyl)-2-oxo-2,4,5,6,7,7a-hexahydrothieno[3,2-c ]pyridine,
2-acetoxy-5-(α-methoxycarbonyl-2-fluorobenzyl)-4,5,6,7-tetrahydrothieno[3,2-c] pyridine,
5-(2-chlorobenzyl)-4,5,6,7-tetrahydrothieno[3,2-c]pyridine (nonproprietary name: ticlopidine; available as ticlopidine hydrochloride),
5-(α-methoxycarbonyl-2-chlorobenzyl)-4,5,6,7-tetrahydrothieno[3,2-c]pyridine (nonproprietary name: cropidogrel; available as clopidogrel sulfate),
5-(α-methoxycarbonyl-2-fluorobenzyl)-4,5,6,7-tetrahydrothieno[3,2-c]pyridine,
5-(α-cyclopropylcarbonyl-2-chlorobenzyl)-4,5,6,7-tetrahydrothieno [3,2-c]pyridin e,
5-(α-cyclopropylcarbonyl-2-fluorobenzyl)-4,5,6,7-tetrahydrothieno[3,2-c]pyridin e,
5-(α-propionyl-2-chlorobenzyl)-4,5,6,7-tetrahydrothieno[3,2-c]pyridine, and
5-(α-propionyl-2-fluorobenzyl)-4,5,6,7-tetrahydrothieno[3,2-c]pyridine; and salts thereof.

In the present invention, the drug having an unpleasant taste is preferably ofloxacin, levofloxacin, sitafloxacin hydrate, cetraxate hydrochloride, nefiracetam, ticlopidine hydrochloride or clopidogrel sulfate.

Examples of the wax (specifically, a wax having a melting point of 40-150°C) which is used in the present invention include fats and oils such as hydrogenated oils (e.g., hydrogenated castor oil, hydrogenated soybean oil, hydrogenated rape seed oil, hydrogenated cotton seed oil) and fats and oils of vegetable or animal origin (e.g., carnauba wax, white beeswax, beef tallow); alcohols and polyhydric alcohols such as higher alcohols (e.g., stearyl alcohol, cetanol) and polyethylene glycol (e.g., macrogol 4000, macrogol 6000); fatty acids and derivatives thereof such as higher fatty acids (e.g., stearic acid, parmitic acid) and fatty acid glycerin esters and fatty acid sucrose esters (e.g., mono-fatty acid glycerin ester, tri-fatty acid glycerin ester); and mixtures of two or more of these substances. Of these, hydrogenated oils, fatty acids, and derivatives of fatty acids are preferred; with hydrogenated oils, higher fatty acids, and fatty acid esters being more preferred; and hydrogenated oils, mono-fatty acid glycerin esters, tri-fatty acid glycerin esters, and stearic acid being particularly preferred. From the viewpoint of the effect of masking the disagreeable taste of the drug, the wax preferably has a melting point lower than that of the drug.

In the present invention, there may preferably be used sugar alcohols having low heat of dissolution; for example, erythritol, xylitol, sorbitol, maltitol, or a mixture of two or more of these compounds. From the viewpoint of sensation upon oral administration, a sugar alcohol having a heat of dissolution of -30 cal/g or lower is preferred, and erythritol and xylitol are particularly preferred.

In the present invention, from the viewpoints of solubility and the effect of masking the disagreeable taste, the weight ratio of the drug having a disagreeable taste to wax is preferably in the range of between 1:1 and 1:5, more preferably between 1:2 and 1:3. The percentage of sugar alcohol in the mixture is preferably 10 wt.% or higher, more specifically 10-99.9 wt.%, more preferably 20-80 wt.%, most preferably 30-70 wt.%.

The granular composition in the present invention may be prepared as follows. A wax is melted with heat, and the drug having a disagreeable taste is dispersed or dissolved therein. Subsequently, the resultant dispersion or solution is subjected to primary granulation to thereby obtain granules. The granules are mixed with sugar alcohol, or the granules are further subjected to secondary granulation.

Primary granulation may be performed through spray granulation or melting granulation. Alternatively, a solution may be cooled to solidification, followed by crushing. Spray granulation is preferred. Particularly, spray-chilling and spray-drying are preferred, because these methods can easily yield fine particles, causing no disagreeable, foreign sensation to the mouth. The size of granules preferably falls within the range of 50-200 µm, particularly 80-120 µm.

When spray granulation is performed for primary granulation, a small quantity of a surfactant may be added for reducing the adhesion of granules to inner walls of a manufacturing apparatus during the spray-chilling process. The quantity of the surfactant may preferably be in the range of 0.5-5 wt.%, particularly preferably 1-4 wt.% or thereabouts.

Secondary granulation of sugar alcohol and granules prepared through primary granulation may be accomplished by wet fluidized bed granulation, wherein a binder solution such as a solution of hydroxypropylcellulose, hydroxypropylmethylcellulose, or polyvinylpyrrolidone is used. Alternatively, secondary granulation may be accomplished by hot-melt granulation, wherein a low-melting-point substance such as polyethyleneglycol or glycerin monostearate is used as a binder.

The granular pharmaceutical composition of the present invention is preferably prepared by any one of the above-mentioned methods. Briefly, through primary granulation, there can be formed granules in which the drug is dispersed uniformly in a wax, to thereby achieve successful masking of the disagreeable taste, because of very low solubility of the wax in the mouth. In the mouth, sugar alcohol is dissolved in saliva in approximately ten seconds, leaving the wax containing the drug in the form of a dispersion. However, since particles of the waxy substance are fine spheres, they provide no disagreeable, gritty taste to the mouth. Sugar alcohols, particularly erythritol and xylitol, taste sweet and deliver fresh and cool sensation to the mouth, yielding the effect of masking the drug's disagreeable taste. After being swallowed, the wax releases the drug in the digestive tract, resulting in absorption of the drug into the body.

The granular pharmaceutical composition in the present invention may be prepared―with or without addition of other additives according to needs―into pharmaceutical products for oral administration, such as powder, granules, dry syrups, tablets, and capsules. Particularly, powder, granules, and dry syrups are preferred.

Examples of the additives used for the above-mentioned formulation may include a binder such as polyvinylpyrrolidone, polyvinylalcohol, hydroxypropylcellulose, hydroxypropylmethylcellulose, methyl cellulose, polyethyleneglycol, or glycerin monostearate; and a sweetener such as aspartame, saccharin sodium, saccharin, thaumatin, or stevia; aromatic ingredients such as dl-menthol and 1-menthol; fluidizing agents such as light anhydrous silicic acid, magnesium aluminometasilicate, talc, synthetic aluminum silicate, and ethylcellulose; disintegrants such as cross carmellose calcium, starch clacium gluconate, and low-substituted hydroxypropylcellulose; and pH adjustors such as sodium citrate and sodium bicarbonate. The additives contains water-soluble polymers. In the present invention, such additives containing water-soluble polymers are preferably used in small amounts; i.e., they account for 0.1-5% by weight, particularly preferably 1-4% by weight, in the pharmaceutical composition.

### Examples

The present invention will next be described in more detail by way of examples, which should not be construed as limiting the invention thereto.

### Example 1

Glycerin monostearate (200 parts by weight) was melted at 90°C, and levofloxacin (100 parts by weight) was uniformly dispersed therein. The dispersion was spray-chilled by use of a spray drier to thereby obtain minute granules. Erythritol (630 parts by weight) was added to the granules (300 parts by weight) and the mixture was mixed by use of a fluidized-bed granulator. Subsequently, polyvinyl aqueous alcohol solution (10 w/v%) in an amount equivalent to 10 parts by weight of polyvinyl alcohol was sprayed onto the mixture for fluidized-bed granulation. After spraying, the granules were dried in the fluidized-bed granulator. The resultant granules were sieved by use of a No. 30 sieve (mesh size: 500 µm) to thereby obtain a powder.

### Example 2

Glycerin monostearate (197 parts by weight) was melted at 90°C, and polyoxyethylene(20)sorbitan monooleate (polysorbate 80) (3 parts by weight) was added thereto. Levofloxacin (100 parts by weight) was uniformly dispersed in the resultant mixture. The dispersion was spray-chilled by use of a spray drier to thereby obtain minute granules. Erythritol (630 parts by weight) was added to the granules (300 parts by weight), followed by mixing by use of a fluidized-bed granulator. Subsequently, polyvinyl alcohol aqueous solution (10 w/v%) in an amount equivalent to 20 parts by weight of polyvinyl alcohol was sprayed onto the mixture for fluidized-bed granulation. After spraying, the granules were dried in the fluidized-bed granulator. The resultant granules were sieved by use of a No. 30 sieve (mesh size: 500 µm) to thereby obtain a powder.

In a manner similar to that described in Examples 1 and 2, powder products were prepared from ofloxacin, sitafloxacin hydrate, cetraxate hydrochloride, or nefiracetam.

### Test Example 1 (Evaluation of effect of masking disagreeable taste: Sensory test 1)

The powder (940 mg) obtained in Example 1 and the powder (950 mg) obtained in Example 2 were subjected to a sensory test. Each of the powders was actually put in the mouth in an amount equivalent to 100 mg of levofloxacin, and the taste and the sensation were evaluated. The two powders were found to have masked the very disagreeable taste of the drug for more than 30 seconds. After elapse of 10 seconds following ingestion, no foreign sensation was felt in the mouth.

### Test Example 2 (Evaluation of effect of masking disagreeable taste: Dissolution test 1)

The powder (940 mg) obtained in Example 1 and the powder (950 mg) obtained in Example 2 were subjected to a test for evaluating the effect of unmasking a disagreeable taste, which was conducted by use of an dissolution test apparatus (test fluid: 500 ml of purified water; temperature of the test fluid 37°C; paddle method; rotating speed: 100 rpm). Use of the drug alone served as a control. The results are shown in Table 1. The dissolution of the drug at the initial stage was significantly suppressed as compared with the case in which the drug was used alone.

**Table 1**

| Results of elution test | | | | |
|---|---|---|---|---|
| Time (seconds) | 10 | 20 | 30 | 60 |
| Drug alone | 58 | 83 | 93 | 103 |
| Example 1 | 2 | 6 | 12 | 29 |
| Example 2 | 5 | 12 | 19 | 40 |

### Test Example 3 (Evaluation of adaptability to administration using tube- 1)

The powders obtained in Examples 1 and 2 were evaluated for their adaptability to use in per tubam administration. Each of the powders (950 mg) was dispersed in purified water (20 ml). The dispersion was placed in a disposable syringe, which was connected to an enteral feeding tube (trade name: ARGAIL, manufactured by Japan Sharwood; new enteral feeding tube, internal diameter 1.0 mm). The dispersion was extruded from the syringe, and the top end of the syringe and the top end of the tube were checked for clogging. The results are shown in Table 2.

**Table 2**

| Results of evaluation of adaptability to administration using tube | |
|---|---|
| | Results |
| Example 1 | No clogging was observed at the top end of the syringe or the top end of the tube |
| Example 2 | No clogging was observed at the top end of the syringe or the top end of the tube |

In Examples 1 and 2, no clogging occurred, and thus, smooth administration was found possible.

### Test Example 4 (Dissolution Test 1)

The powder (940 mg) obtained in Example 1 and the powder (950 mg) obtained in Example 2 were subjected to a dissolution test, which was conducted by use of a dissolution test apparatus (test fluid: 900 ml of a first fluid as described in the Pharmacopoeia of Japan, disintegration test; temperature of the test fluid: 37°C; paddle method; rotating speed: 50 rpm). As is apparent from Table 3, these powders were found to show excellent dissolution.

**Table 3**

| Results of dissolution test | | | | | | |
|---|---|---|---|---|---|---|
| (average dissolution ratio (%)) | | | | | | |
| Time | after 5 min | after 10 min | after 20 min | after 30 min | after 45 min | after 60 min |
| Example 1 | 100 | 100 | 100 | 100 | 100 | 100 |
| Example 2 | 98 | 98 | 98 | 98 | 98 | 98 |

### Example 3

Tri-fatty acid glycerin ester (216 parts by weight) was melted at 80°C, and polyoxyethylene(20)sorbitan monooleate (polysorbate 80) (11.2 parts by weight) was added thereto. Clopidogrel sulfate (97.8 parts by weight) was uniformly dispersed in the resultant mixture. The dispersion was spray-chilled by use of a spray drier to thereby obtain minute granules. Erythritol (169 parts by weight) and aspartame (5 parts by weight) were added to the granules (325 parts by weight) to thereby obtain powder.

### Example 4

Tri-fatty acid glycerin ester (216 parts by weight) was melted at 80°C, and polyoxyethylene(20)sorbitan monooleate (polysorbate 80) (11.2 parts by weight) was added thereto. Clopidogrel sulfate (97.8 parts by weight) was uniformly dispersed in the resultant mixture. The dispersion was spray-chilled by use of a spray drier to thereby obtain minute granules. Erythritol (169 parts by weight) was added to the granules (325 parts by weight), followed by mixing by use of a fluidized-bed granulator. Subsequently, polyvinyl alcohol aqueous solution (10 w/v%) in an amount equivalent to 20 parts by weight of polyvinyl alcohol was sprayed onto the mixture for fluidized-bed granulation. After spraying, the granules were dried in the fluidized-bed granulator. The resultant granules (514 parts by weight) were mixed with aspartame (5 parts by weight) to thereby obtain powder.

### Example 5

Tri-fatty acid glycerin ester (216 parts by weight) was dissolved into dichloromethane. Clopidogrel sulfate (97.8 parts by weight) and ethylcellulose (32.6 parts by weight) were uniformly dispersed in the resultant mixture. The dispersion was spray-chilled by use of a spray drier to thereby obtain minute granules. Erythritol (147.6 parts by weight) and aspartame (5 parts by weight) were added to the granules (346.4 parts by weight) to thereby obtain powder.

### Example 6

Tri-fatty acid glycerin ester (216 parts by weight) was dissolved into dichloromethane. Clopidogrel sulfate (97.8 parts by weight) and ethylcellulose (32.6 parts by weight) were uniformly dispersed in the resultant mixture. The dispersion was spray-chilled by use of a spray drier to thereby obtain minute granules. Erythritol (147.6 parts by weight) was added to the granules (346.4 parts by weight), followed by mixing by use of a fluidized-bed granulator. Subsequently, polyvinyl alcohol aqueous solution (10 w/v%) in an amount equivalent to 20 parts by weight of polyvinyl alcohol was sprayed onto the mixture, for fluidized-bed granulation. After spraying, the granules were dried in the fluidized-bed granulator. The resultant granules (514 parts by weight) were mixed with aspartame (5 parts by weight) to thereby obtain powder.

### Comparative Example 1

Tri-fatty acid glycerin ester (135 parts by weight) was melted at 80°C, and polyoxyethylene(20)sorbitan monooleate (polysorbate 80) (7 parts by weight) was added thereto. Clopidogrel sulfate (61 parts by weight) was uniformly dispersed in the resultant mixture. The dispersion was spray-chilled by use of a spray drier to thereby obtain minute granules. Lactose (147.6 parts by weight) and aspartame (5 parts by weight) were added to the granules (346.4 parts by weight) to thereby obtain powder.

### Test Example 5 (Evaluation of effect of masking disagreeable taste: Sensory test 2)

The powders (500 mg) obtained in Examples 3 to 6 were each subjected to a sensory test. Each of the powders was actually put in the mouth in an amount equivalent to 100 mg of clopidogrel sulfate, and the taste and the sensation were evaluated. All of theese powders were found to have masked the very disagreeable taste of the drug for more than 30 seconds. After elapse of 10 seconds following ingestion, no foreign sensation was felt in the mouth.

### Test Example 6 (Evaluation of effect of masking disagreeable taste: Dissolution test 2)

The powders (500 mg) obtained in Examples 3 to 6 were subjected to a test for evaluating the effect of unmasking a disagreeable taste, which was conducted by use of a dissolution test apparatus (test fluid: 300 ml of purified water; temperature of the test fluid 37°C; paddle method; rotating speed: 100 rpm). As a result, it was confirmed that each powder is able to significantly suppress the dissolution of the drug at the initial stage, as compared with the case in which the drug was used alone.

### Test Example 7 (Evaluation of adaptability to administration using tube- 2)

The powders obtained in Comparative Example 1 and Example 5 were evaluated for their adaptability to use in administration using tube. Each of the powders (500 mg) was dispersed in purified water (20 ml). The dispersion was placed in a disposable syringe, which was connected to an enteral feeding tube (trade name: ARGAIL, manufactured by Japan Sharwood; new enteral feeding tube, internal diameter 1.0 mm). The dispersion was extruded from the syringe, and the top end of the syringe and the top end of the tube were checked for clogging. The results are shown in Table 4.

**Table 4**

| Results of evaluation of adaptability to administration using tube | |
|---|---|
| | Results |
| Comparative Example 1 | Clogging was observed at the top end of the tube immediately after pushing of the powder and the dispersion was hardly pushed out from the tube. |
| Example 5 | No clogging was observed at the top end of the syringe or the top end of the tube |

The result of comparative Example 1 shows that this comparative product is too difficult to be effectively administered and therefore is not suited to administration using tube. By contrast, the product of Example 5 causes no undesired clogging, thereby making it possible to be smoothly administered.

### Test Example 8 (Dissolution Test 2)

The powder (326.5 mg) obtained in Example 3 was subjected to an elution test, which was conducted by use of an elution test apparatus (test fluid: 900 ml of a first fluid as described in the Pharmacopoeia of Japan (contaning sodium lauryl sulfate at 1%), disintegration test; temperature of the test fluid: 37°C; paddle method; rotating speed: 50 rpm). As shown in Table 5, the powder of Example 3 was found to show excellent dissolution.

**Table 5**

| Results of elution test | | | | | | |
|---|---|---|---|---|---|---|
| Time | after 10 min | after 15 min | after 20 min | after 30 min | after 45 min | after 60 min |
| Average elution ratio (%) | 75.7 | 83.4 | 88.5 | 94.0 | 99.7 | 100.9 |

### Industrial Applicability of the Invention

The present invention provides a pharmaceutical product which excellently masks a disagreeable taste of a drug and which provides favorable sensation upon oral administration and therefore is easily ingested by even the elderly, children, and patients suffering dysphagia. This pharmaceutical product is also suitable for administration using tube.

## Claims

1. A granular pharmaceutical composition comprising a drug having a disagreeable taste, a wax, and a sugar alcohol.

2. A granular pharmaceutical composition according to claim 1, which comprises a granular material containing the drug having a disagreeable taste and the wax, and the sugar alcohol.

3. A granular pharmaceutical composition according to claim 1 or 2, wherein the drug having a disagreeable taste is slightly soluble in the wax.

4. A granular pharmaceutical composition according to claim 1 or 2, wherein the drug having a disagreeable taste is soluble in water and slightly soluble in the wax.

5. A granular pharmaceutical composition according to any one of claims 1 through 4, wherein the wax has a melting point of 40-150°C.

6. A granular pharmaceutical composition according to any one of claims 1 through 5, wherein the wax is a member selected from the group consisting of hydrogenated oils, fats and oils of vegetable or animal origin, higher alcohols, polyethylene glycols, higher fatty acids, glycerin fatty acid esters, sucrose fatty acid esters, and combinations of two or more of these.

7. A granular pharmaceutical composition according to any one of claims 1 through 6, wherein the sugar alcohol is a member selected from the group consisting of erythritol, xylitol, sorbitol, maltitol, and combinations of two or more of these.

8. A granular pharmaceutical composition according to any one of claims 1 through 7, wherein the sugar alcohol has a heat of dissolution of not higher than -30 cal/g.

9. A granular pharmaceutical composition according to any one of claims 1 through 8, wherein the sugar alcohol is erythritol and/or xylitol.

10. A granular pharmaceutical composition according to any one of claims 1 through 9, wherein the drug having a disagreeable taste is a drug selected from the group consisting of cetraxate hydrochloride, ecapapide, nefiracetam, talampicillin hydrochloride, indenolol hydrochloride, hydralazine hydrochloride, chlorpromazine hydrochloride, tiaramide hydrochloride, berberine chloride, digitoxin, sulpyrine, azelastine hydrochloride, etilefrine hydrochloride, diltiazem hydrochloride, propranolol hydrochloride, chloramphenicol, aminophyllin, erythromycin, clarithromycin, phenobarbital, calcium pantathenate, indeloxazine hydrochloride, aminoguanidine hydrochloride, bifemelane hydrochloride, 7β-[2-(2-aminothiazol-4-yl)-2-(Z)-hydroxyiminoacetamido]-3-N,N-dimethylc arbamoyloxymethyl-3-cephem-carboxylic acid 1-(isopropoxycarbonyloxy)ethyl ester hydrochloride, (E)-3-(2-methoxy-3,6-dimethyl-1,4-benzoquinon-5-yl)-2-[5-(3-pyridyl)pentyl ]-2-propenic acid, aminophylline, theophylline, diphenhydramine, metoclopramide, phenylbutazone, phenobarbital, ampicillin, cimetidine, famotidine, nizatidine, acetaminophen, epirizole, pyrazinamide, caffeine, ethionamide, carvedilol, ranitidine hydrochloride, roxatidine acetate hydrochloride, imipramine hydrochloride, ephedrine hydrochloride, diphenhydramine hydrochloride, tetracycline hydrochloride, doxycycline hydrochloride, naphazoline hydrochloride, noscapine hydrochloride, papaverine hydrochloride, dextrhomethorphan hydrobromide, timepidium bromide, chlorphenilammonium maleate, alimemazine tartrate, pilsicainide hydrochloride, N-methylscopolamine methylsulfate, cinepazide maleate, arginine hydrochloride, histidine hydrochloride, lysine hydrochlroride, lysine acetate; crude drugs or extracts thereof; pyrridonecarboxylic acid compounds represented by formulas (1) through (4) and salts thereof: 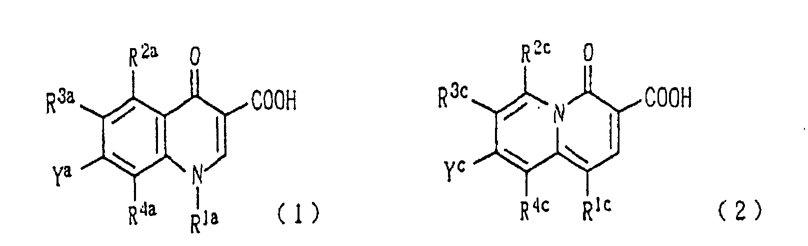 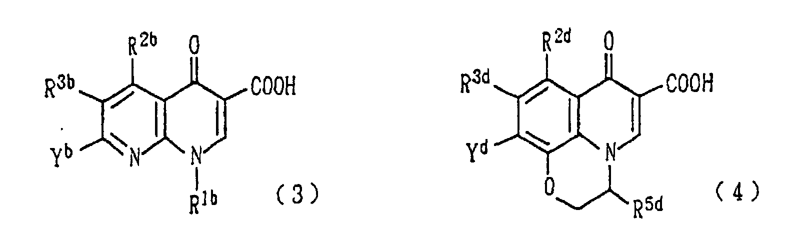
(wherein each of R^{1a}, R^{1b}, and R^{1c} represents a C1-C6 linear or branched alkyl group which may have a substituent, a C3-C6 cyclic alkyl group which may have a substituent, an aryl group which may have a substituent, or a heteroaryl group which may have a substituent;
each of R^{2a}, R^{2b}, R^{2c}, and R^{2d} represents a hydrogen atom or a C1-C6 linear or branched alkyl group which may have a substituent; or an amino group each of R^{3a}, R^{3b}, R^{3c}, and R^{3d} represents a hydrogen atom or a halogen atom; R^{4a} or R^{4c} represents a hydrogen atom, a halogen atom, a C1-C6 linear or branched alkyl group which may have a substituent; or a C1-C6 linear or branched alkoxyl group which may have a substituent;
R^{5d} represents a hydrogen atom or a C1-C6 linear or branched alkyl group which may have a substituent; and
each of Y^{a}, Y^{b}, Y^{c}, and Y^{d} represents a nitrogen-containing group).

11. A granular pharmaceutical composition according to any one of claims 1 through 9, wherein the drug having a disagreeable taste is ofloxacin.

12. A granular pharmaceutical composition according to any one of claims 1 through 9, wherein the drug having a disagreeable taste is levofloxacin.

13. A granular pharmaceutical composition according to any one of claims 1 through 9, wherein the drug having a disagreeable taste is clopidogrel sulfate

14. A granular pharmaceutical composition according to any one of claims 1 through 13, wherein the drug having a disagreeable taste and the wax are mixed at a ratio of 1:1 - 1:5 by weight, and the composition has a sugar alcohol content of at least 10% by weight.

15. A granular pharmaceutical composition according to any one of claims 1 through 14, which is produced by melting the wax with heat; dispersing or dissolving therein the drug having a disagreeable taste; subjecting the resultant mixture to primary granulation to thereby obtain a granulated product; and mixing the granulated product with the sugar alcohol, or subjecting the granulated product and the sugar alcohol to secondary granulation.

16. A granular pharmaceutical composition according to claim 15, wherein the primary granulation is spray granulation.

17. A granular pharmaceutical composition according to claim 15 or 16, wherein the particle size of a particle resulting from the primary granulation is 50-200 µm.

18. A method of producing a granular pharmaceutical composition, which method comprises melting the wax with heat; dispersing or dissolving a drug having a disagreeable taste therein; subjecting the resultant mixture to primary granulation to thereby obtain a granulated product; and mixing the granulated product with the sugar alcohol or subjecting the granulated product and the sugar alcohol to secondary granulation.

19. A pharmaceutical product for oral administration comprising a granular pharmaceutical composition as recited in any one of claims 1 through 17.

20. A pharmaceutical product for oral administration according to claim 19, which has a dosage form of powder or granules.
